# FASCICULE DE BREVET EUROPEEN

(11) **EP 2 821 048 B1**
(45) Date de publication et mention de la délivrance du brevet: **07.08.2019**
(21) Numéro de dépôt: 14173969.8
(22) Date de dépôt: 25.06.2014
(51) Int. Cl.: A61H 7/00, A61N 5/06

(54) **Appareil de soin avec montage de tête facile**
Pflegegerät mit leichter Montage des Gerätekopfs
Care apparatus with easy head mounting

(30) Priorité: 04.07.2013 FR 1356566
(43) Date de publication de la demande: 07.01.2015
(73) Titulaire: SEB S.A., 69130 Ecully (FR)
(72) Inventeur: Giraud, Camille, 69001 LYON (FR); Mandica, Franck, 69340 FRANCHEVILLE (FR)
(74) Mandataire: Guéry-Jacques, Géraldine

(56) Documents cités:
- EP-A1- 0 102 667
- CH-A- 332 392
- CN-Y- 2 150 025
- FR-A- 1 091 761
- FR-A- 1 447 039
- JP-U- S54 135 297
- US-A- 2 343 553

## Description

La présente invention concerne le domaine des appareils de soin pour la peau. L'invention concerne plus particulièrement un appareil de soin comprenant au moins une tête de massage détachable du boîtier de l'appareil, ladite tête de massage étant destinée à être mise en mouvement par un moteur électrique intégré au boîtier.

On connait dans ce domaine un document US 4 919 117 décrivant un appareil de traitement corporel destiné à nettoyer et exfolier le visage ou le corps à l'aide de plusieurs embouts de nettoyage ou d'exfoliation interchangeables. Chaque embout est entrainé rotatif autour d'un axe pour que l'applicateur fixé à la surface de contact puisse suivre un trajet circulaire ou en ellipse. L'applicateur peut être des poils de brosse, un savon cosmétique ou encore une pierre ponce. Chaque embout est solidaire d'un arbre identique destiné à s'emboîter dans le système d'entraînement. Les traitements proposés par l'appareil nécessitent une vitesse de rotation très importante. Ainsi, cet appareil n'est pas compatible avec un embout de massage destiné à tourner à une vitesse réduite pour le confort de l'utilisateur.

Le document CN2150025Y décrit un rasoir électrique qui peut être équipé d'une tête de massage amovible. Ce document divulgue un appareil de soin pour la peau comprenant un corps creux formant une zone de préhension, l'appareil étant prévu pour recevoir de façon détachable au moins une tête de massage ledit corps creux comprenant un moteur électrique destiné à entraîner un moyen d'entraînement autour d'un axe par l'intermédiaire d'un moyen de transmission mécanique, ladite au moins une tête de massage étant destinée à être actionnée par ledit moyen d'entraînement.

Le document EP 0 102 667 décrit un rasoir électrique équipé d'un accouplement entre le moteur et les lames particulièrement compact. Cet appareil de soin pour la peau comprend un corps creux formant une zone de préhension et un logement prévu pour recevoir de façon détachable au moins un élément de coupe, ledit corps creux comprenant un moteur électrique, destine a entraîner un moyen d'entraînement autour d'un axe par l'intermédiaire d'un moyen de transmission mécanique , ledit au moins un élément de coupe étant destine a être actionne par ledit moyen d'entraînement. Le moyen de transmission mécanique comprend un moyen de guidage dudit moyen d'entraînement en translation selon ledit axe entre au moins deux positions distinctes.

Un autre document EP489212 décrit un appareil de massage pour la peau comprenant deux têtes amovibles et interchangeables entre elles, chacune des têtes présentant des moyens de massage en protubérance destinés à masser la peau pendant la rotation de ladite tête. L'appareil comprend un arbre d'entrainement à section polygonale intégré au corps destiné à s'emboîter dans une cavité ayant une forme correspondante dans la tête pour l'entrainement de celle-ci lorsque la tête est montée sur le corps. Ce document propose donc un appareil de massage motorisé à têtes interchangeables.

Partant de ce document, l'inventeur était confronté à un nouveau problème :développer un appareil comprenant des éléments mécaniques destinés à être mis en mouvement à des vitesses ou fréquences faibles, quelques rotations par minute, soit quelques hertz. Le moteur utilisé doit donc être associé à une forte réduction (par exemple, un motoréducteur). Du fait que l'arbre d'entrainement est lié en rotation au moteur via un couple élevé, il peut difficilement tourner sur lui-même quand le moteur est en arrêt. De plus, une seule position est souhaitée via un indexage, dans le but d'améliorer l'utilisation de l'appareil et son ergonomie en privilégiant une orientation de la tête vis-à-vis de la peau. L'utilisateur sera amené à tourner la tête choisie et à trouver progressivement la bonne position permettant le montage, ce qui complique l'utilisation de l'appareil.

Le but de l'invention est de remédier aux inconvénients susmentionnés et de proposer un appareil de soin pour la peau motorisé ayant un montage de tête facile, rapide et intuitif.

Un autre but de l'invention est de proposer un appareil de soin pour la peau dont au moins une tête peut être montée selon une unique position de fonctionnement et dont le montage dans cette position est intuitif.

Un autre but de l'invention est de proposer un appareil de soin pour la peau muni d'un système d'entrainement qui ne tourne pas facilement et dont le montage de la tête sur ce système ne demande pas trop de force.

Un autre but de l'invention est de proposer un appareil de soin pour la peau dont au moins une tête peut être démontée sans détériorer le moteur et les éléments mécaniques de la tête et du corps creux.

Un autre but de l'invention est de proposer un appareil de soin pour la peau dont au moins une tête peut être montée de façon guidée et progressive.

Un autre but de l'invention est de proposer un appareil de soin pour la peau compact et économique.

Encore un autre but de l'invention est de proposer un appareil de soin pour la peau plus silencieux et plus agréable à l'utilisation.

Encore un autre but de l'invention est de proposer un appareil de soin pour la peau ayant un rendu qualitatif.

Encore un autre but de l'invention est de proposer un appareil de soin pour la peau du visage, notamment les couches supérieures de l'épiderme, pour traiter les signes de vieillissement comme les rides et/ou donner un coup d'éclat et/ou aider la peau à un meilleur accueil d'un produit cosmétique à appliquer avant, pendant ou après le massage.

Ces buts sont atteints avec un appareil de soin pour la peau comprenant un corps creux formant une zone de préhension et un logement prévu pour recevoir de façon détachable au moins une tête de massage; ledit corps creux comprenant un moteur électrique destiné à entrainer un moyen d'entrainement autour d'un axe Δ par l'intermédiaire d'un moyen de transmission mécanique ; ladite au moins une tête de massage étant destinée à être actionnée par ledit moyen d'entrainement. Le moyen de transmission mécanique comprend un moyen de guidage dudit moyen d'entrainement en translation selon ledit axe Δ entre au moins deux positions distinctes, dont une position de marche (PM) et une position escamotée (PE). Le moyen d'entrainement rétracté dans le corps creux de l'appareil permet une fixation correcte de la tête de massage par rapport au corps creux. On aura donc la possibilité d'ajouter des moyens d'indexage pour faciliter le montage et privilégier une position de la tête de massage par rapport au corps creux.

Avantageusement, l'appareil de soin comprend un moyen de rappel actif entre le moyen d'entrainement et le moyen de guidage destiné à ramener le moyen d'entrainement en position de marche (PM). Le moyen de rappel, par exemple un ressort compressé, permet de pousser le moyen d'entrainement vers la tête pour la bonne mise en place.

De surcroît, ledit moyen d'entrainement présente une extrémité entrainante destinée à entrer en contact avec ladite tête de massage et une extrémité entrainée destinée à rester en contact avec ledit moyen de guidage.

Avantageusement, la tête de massage comprend une connexion mécanique présentant une forme complémentaire à la forme de ladite extrémité entrainante. Ceci permet une liaison solide entre la tête de massage et le moyen d'entrainement. Cette forme peut varier en fonction du moulage, telle qu'une forme en croix, en carré ou en étoile.

De préférence, ladite extrémité entrainante présente une forme saillante par rapport au moyen d'entrainement et la connexion mécanique présente une forme creuse par rapport à la tête de massage. Ceci permet que la tête de massage soit plus compacte et plus légère. De plus, en l'absence de partie saillante, elle a moins de risque d'être détériorée pendant le transport.

Selon un deuxième de mode de réalisation, l'extrémité entrainée présente une forme substantiellement cylindrique et comprend sur la surface latérale au moins un plot destiné à se déplacer entre la position de marche (PM) et la position escamotée (PE) sur au moins une rampe de guidage appartenant au moyen de guidage.

Dans ce deuxième mode de réalisation, le moyen de guidage comprend au moins une cale destinée à entrer en contact avec ledit plot et à pouvoir entrainer celui-ci en rotation dans un sens comme dans l'autre lorsque le moyen d'entrainement est en position de marche (PM). Le deuxième mode de réalisation permet que le bruit éventuel dû à l'enclenchement du moyen d'entrainement dans la tête de massage survienne, non au moment de la mise en marche du moteur, mais au moment de la mise en place de la tête de massage sur le corps creux, ce qui paraitra plus logique à l'utilisateur et augmentera le confort d'utilisation de l'appareil.

Avantageusement, l'appareil de soin comprend des moyens d'indexage entre la tête de massage et le corps creux, pour faciliter le montage de la tête de massage et limiter celle-ci dans une seule position possible.

De surcroît, l'appareil de soin comprend des moyens de fixation, par clips ou grâce à un bouton avec ressort, entre la tête de massage et le corps creux pour un montage et démontage de la tête de massage plus faciles.

Afin d'atténuer le bruit lors de la mise en place de la tête de massage, l'appareil comprend un moyen d'amortissement sur l'extrémité entrainante du moyen d'entrainement. Le moyen d'amortissement peut aussi être agencé sur la tête de massage ou sur le corps creux.

Selon un mode préféré de l'invention, le corps creux est d'une forme longitudinale selon l'axe Δ pour que la tête de massage montée se trouve à une extrémité du corps creux, ce qui permet un appareil ergonomique qui est facile à prendre dans une main.

Enfin, l'appareil de soin peut comprendre au moins une source lumineuse destinée à irradier la peau.

L'invention sera mieux comprise à l'étude des modes de réalisation pris à titre nullement limitatif et illustrés dans les figures annexées dans lesquelles :
- La Figure 1 est une vue d'un appareil complet avec trois têtes de massage, détachées du corps de l'appareil ;
- La Figure 2 est une vue d'une tête de massage;
- La Figure 3 est une vue d'une tête de massage identique à celle de la Figure 2 ;
- La Figure 4 est une vue d'un exemple du montage d'une tête de massage sur le corps de l'appareil ;
- La Figure 5 est une vue en perspective du corps de l'appareil ;
- Les Figures 6 et 7 sont des vues de face du corps de l'appareil illustrant le moyen d'entrainement dans deux positions différentes, selon un premier mode de réalisation du guidage du moyen d'entrainement ;
- La Figure 8 est un schéma d'une première variante du moyen d'entrainement et du moyen de guidage ainsi que de l'agencement du moyen de rappel, selon le premier mode de réalisation du guidage du moyen d'entrainement ;
- Les Figures 9 et 10 sont des vues en perspective d'une deuxième variante du moyen d'entrainement et du moyen de guidage, selon le premier mode de réalisation du guidage du moyen d'entrainement ;
- Les Figures 11, 12 et 13 sont des vues en perspective de différentes alternatives du moyen d'entrainement ;
- Les Figures 14 et 15 sont des schémas d'amélioration de forme sur le moyen d'entrainement ;
- Les Figures 16, 17, 18, 19 et 20 sont des schémas illustrant un second mode de réalisation du guidage du moyen d'entrainement.

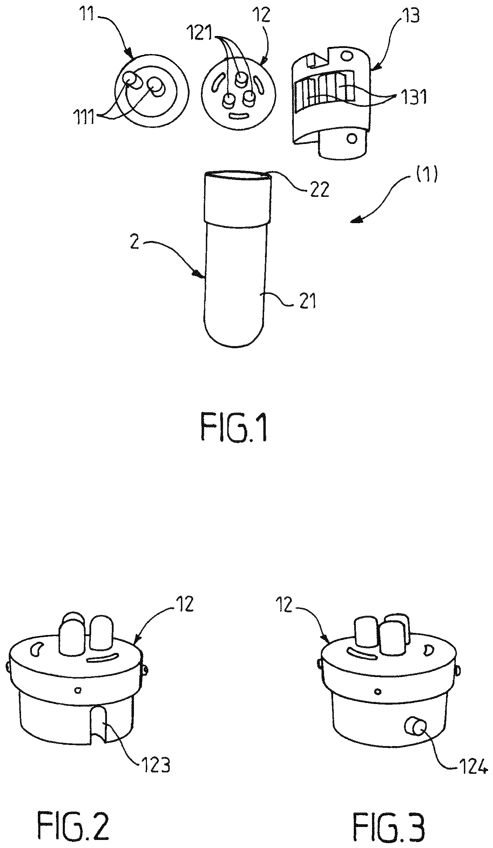

Un appareil de soin pour la peau tel qu'illustré à la Figure 1 et désigné dans son ensemble par la référence 1 comprend un corps creux 2 qui est un boîtier d'une forme longitudinale selon un axe Δ et formant une zone de préhension 21 et un logement 22 destiné à recevoir une des trois têtes de massage 11, 12 et 13. Les têtes de massage illustrées strictement à titre d'exemples comprennent une tête à embouts oscillants 11, une tête à embouts rotatifs 12 et enfin une tête à deux rouleaux 13. Les têtes 11 et 12 sont montées sur le corps creux 2 en ayant des embouts 111, 121 sensiblement parallèles à l'axe Δ, ce qui permet un effort d'appui perpendiculaire à la peau et un massage efficace en profondeur. La tête 13 est montée sur le corps creux 2 avec l'axe de rotation de chacun des rouleaux 131 parallèle à l'axe Δ. Ceci permet d'avoir une zone de traitement plus large et une meilleure ergonomie de l'appareil. Toutefois, tout type de tête de massage peut être envisagé tant que celle-ci comprend un mécanisme permettant de transformer la rotation en un mouvement des moyens de massage sur la peau.

L'appareil de soin comprenant plusieurs têtes de massage à buts différents permet de proposer, pour chaque personne et dans chaque zone cible précise, le traitement le mieux adapté, tout en ayant un appareil compact et économique. Ceci permet également de combiner les traitements pour obtenir un soin complet. Afin d'améliorer l'usage alternatif des têtes, l'appareil comprend une fixation entre la tête et le corps creux permettant un montage et démontage faciles, ainsi qu'un moyen d'indexage pour guider l'utilisateur dans le montage. Pour ce faire, l'appareil de soin comprend des moyens d'indexage 23, 123. Tel que visible aux figures 2, 3 et 5, la tête 12 comprend sur sa surface latérale une rainure 123 destinée à se glisser sur une forme en protubérance 23 dans le logement 22 du corps creux 2. La tête 12 comprend en outre, toujours sur la surface latérale, un bouton 124 monté sur ressort pouvant s'insérer dans un trou traversant 24 sur la paroi du logement 22. Ces moyens de fixation 24, 124 autorisent ainsi une seule position de la tête de massage dans le corps creux, seuls les moyens de massage pouvant être mis en mouvement.

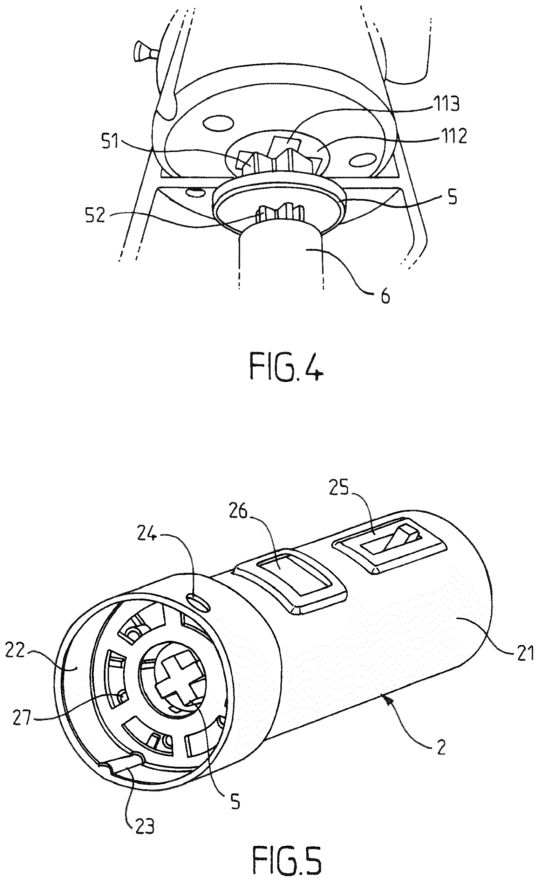

La Figure 4 montre un moyen d'entrainement 5 de la tête de massage, que l'on va appeler « l'entraineur » par la suite. L'entraineur comprend une extrémité entrainante 51 et une extrémité entrainée 52 que l'on expliquera plus loin. Ladite extrémité entrainante 51 présente une forme en croix destinée à s'insérer dans une connexion mécanique 113 présentée sous la forme d'une cavité ayant une forme complémentaire à celle de l'extrémité entrainante 51. La cavité 113 se situe sur un plateau 112 qui est préférentiellement au milieu de la tête de massage 11, 12, ou 13, de façon à ce que l'extrémité entrainante 51 puisse faire tourner le plateau 112 dans un sens ou dans l'autre. Pour chaque tête de massage 11, 12, 13, les moyens de massage sont liés au plateau 112 par un mécanisme (non illustré) afin d'être mis en mouvement. Selon l'invention, la vitesse de rotation de l'entraineur peut être comprise de façon privilégiée entre 30 et 120 rotations par minute.

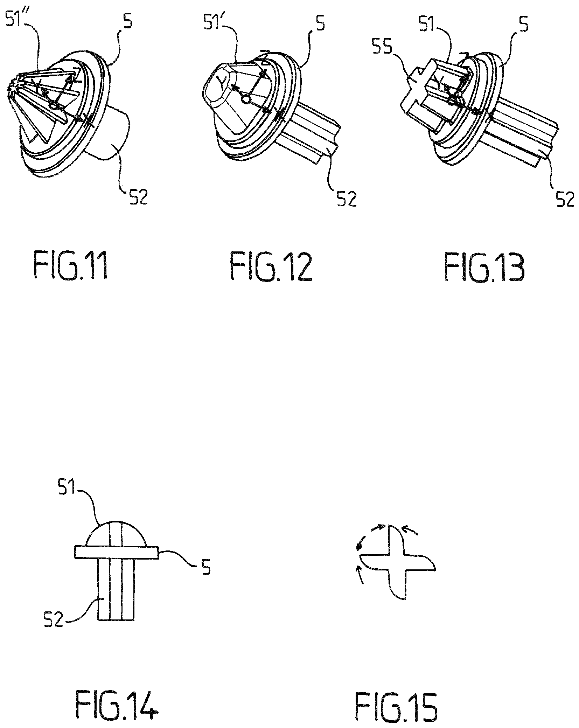

La forme de l'extrémité entrainante 51 et la forme de la cavité 113 peuvent être différentes de celles illustrées à la Figure 4. La Figure 11 montre une extrémité entrainante en étoile 51" et la Figure 12 montre une extrémité en carré 51'. On peut même imaginer une inversion des deux parties, c'est-à-dire, une forme en protubérance sur le plateau 112 et une cavité d'une forme correspondante sur l'extrémité entrainante 51.

Maintenant va être présenté plus précisément le corps creux 2 de l'appareil tel qu'illustré aux Figures 5 à 7. Le corps creux 2 comprend un logement 22, préférentiellement d'une forme cylindrique. Comme montré à la Figure 5, le corps creux peut être pourvu d'au moins une source lumineuse 27, par exemple issue de LED (light emitting diode), en dessous du logement 22 destiné à éclairer et à irradier la peau au travers de la tête de massage 11, 12, 13. Les LED sont destinées à émettre dans une gamme de longueurs d'onde comprises par exemple entre 550 nm et 1600 nm, ce qui correspond au domaine visible du jaune, de l'orange et du rouge, ainsi qu'au domaine du proche infrarouge. Bien sur, la source lumineuse 27 peut être également intégrée à la tête de massage 11, 12, 13 pour être au plus près de la peau. Le corps creux 2 comprend sur la zone de préhension 21 un moyen de commande 25 tel qu'un interrupteur de mise en fonction de l'appareil ou encore un réglage de la vitesse de l'entraineur et/ou de la luminosité de la source lumineuse 27. Un écran 26 est mis à disposition dans cette zone de préhension 21 pour afficher des données liées au fonctionnement de l'appareil.

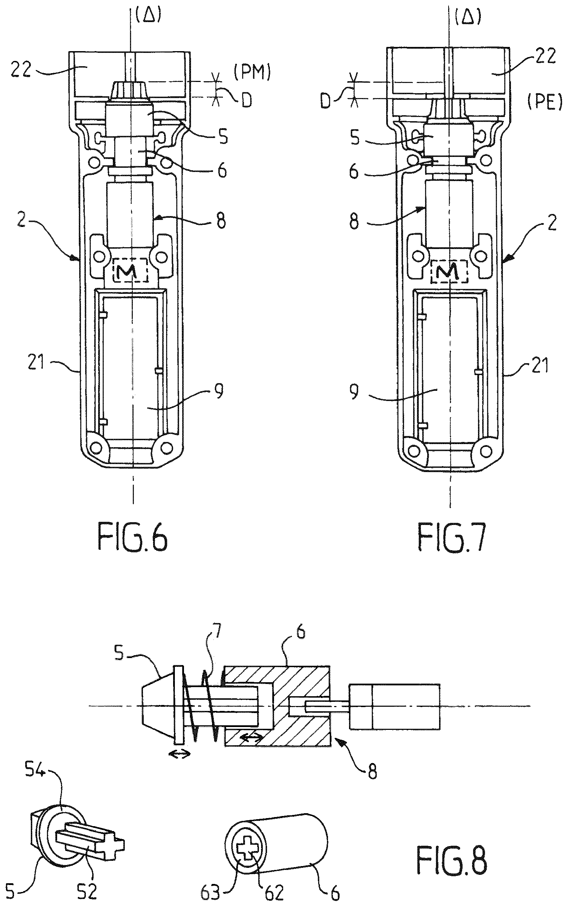

Le corps creux 2 comprend plusieurs éléments mis en série, tel qu'illustrés aux Figures 6 et 7. Il comprend une batterie 9 pour l'alimentation électrique d'un moteur (M). Afin que les têtes de massage 11, 12, 13 puissent fonctionner à une vitesse réduite, le corps creux 2 comprend un moyen de transmission mécanique 8 relié à la sortie du moteur permettant de réduire sa vitesse à un ratio important, par exemple 1/250. Le moyen de transmission mécanique 8 peut comprendre un train d'engrenage formant avec le moteur un motoréducteur. Le moyen de transmission mécanique 8 comprend en outre un moyen de guidage 6 dit l'intermédiaire d'une forme allongée ayant une extrémité solidaire avec la transmission mécanique, par exemple par un montage serré. Dans l'exemple montré à la Figure 8, l'axe du motoréducteur est en forme de méplat et il est inséré dans une cavité sur un coté de l'intermédiaire 6. L'autre extrémité de l'intermédiaire 6 est prévue de telle sorte à pouvoir être liée en rotation avec l'entraineur 5, ainsi l'entraineur 5 est entrainé en rotation autour de l'axe Δ par le moyen de transmission mécanique 8.

Au moment de la mise en place de la tête de massage 11, 12, 13 sur le corps creux 2, il est probable que l'extrémité entrainante 51 et la cavité 113 ne soient pas bien en face. Afin de résoudre ce problème, l'entraineur 5 peut se déplacer sur l'axe Δ vers le moyen de guidage 6 d'une distance D sensiblement égale au dépassement de l'extrémité entrainante 51 dans le logement 22, par exemple d'une valeur entre 3 mm et 15 mm, ce qui permet que le moyen d'entrainement 5 soit complètement rentré dans le corps creux 2 en position escamotée (PE), ne dépasse pas dans le logement 22 et ne gêne ainsi pas la mise en place de la tête de massage. La Figure 6 montre l'entraineur 5 en position de marche (PM) où sensiblement la totalité de l'extrémité entrainante 51 est disponible dans le logement 22 pour recevoir une tête de massage. La Figure 7 montre l'entraineur 5 en position escamotée (PE) où l'extrémité entrainante 51 est rétractée d'une distance D vers l'intérieur du corps creux 2 et elle n'est plus apparente dans le logement 22. Afin de pouvoir ramener l'entraineur 5 en position de marche (PM) pour qu'il soit fonctionnel lorsque la tête de massage est mise en place, un moyen de rappel 7 est actif entre l'entraineur 5 et l'intermédiaire 6. Le moyen de rappel 7 peut être un ressort de compression.

Selon un premier mode de réalisation de l'appareil de soin, tel qu'illustré aux Figures 8, 9 et 10, l'extrémité entrainée 52 de l'entraineur 5 présente une forme en croix destinée à coulisser dans un évidement 62 d'une forme complémentaire de celle de l'extrémité entrainée 52. On peut bien sûr imaginer d'autres formes permettant une liaison glissière entre les deux éléments. Un ressort 7 est disposé entre l'entraineur et l'intermédiaire afin de pousser le premier vers la gauche sur les Figures 8, 9 et 10, vers la position de marche (PM), une rainure sur l'entraineur (54) et une rainure sur l'intermédiaire (63) à l'extérieur de l'évidement 62 étant prévues pour recevoir chaque extrémité du ressort 7.

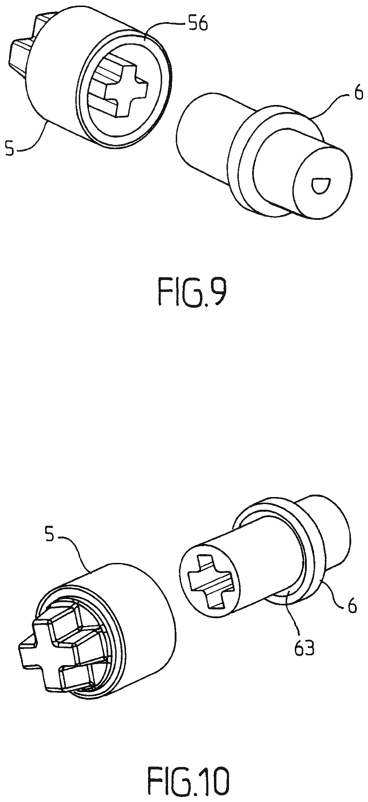

Les Figures 9 et 10 montrent une variante dans laquelle l'entraineur 5 comprend une couronne 56 pouvant couvrir la partie coulissante de l'intermédiaire 6 pour un meilleur guidage, une liaison plus solide et un mouvement plus fluide. D'autres formes de l'extrémité entrainée 52 et de l'intermédiaire 6 peuvent être envisagées et l'agencement du ressort 7 sera amené à s'adapter en fonction des formes choisies. En particulier, il peut se trouver dans l'évidement 62 de l'intermédiaire 6.

En fonctionnement, lors de la mise en place d'une tête de massage 11, 12 ou 13 sur le corps creux 2, l'entraineur 5 est initialement en position de marche (PM). Si les formes complémentaires de l'extrémité entrainante 51 et de la cavité 113 ne sont pas en face, ce qui risque d'arriver le plus souvent, la tête de massage pousse l'entraineur 5 vers la position escamotée (PE) d'une distance D ; une fois que la tête de massage est mise et fixée correctement sur le corps creux 2, l'entraineur 5 arrive en position escamotée (PE) ; on met alors le moteur en marche ce qui fait tourner le moyen de transmission mécanique 8 ainsi que l'entraineur 5 ; il arrive rapidement le moment où les formes complémentaires sont face à face, sous l'effort de poussée du ressort 7 l'entraineur 5 se déplace vers la position de marche (PM) et s'emboîte avec la tête de massage mise en place avant d'entrainer les moyens de massage de celle-ci.

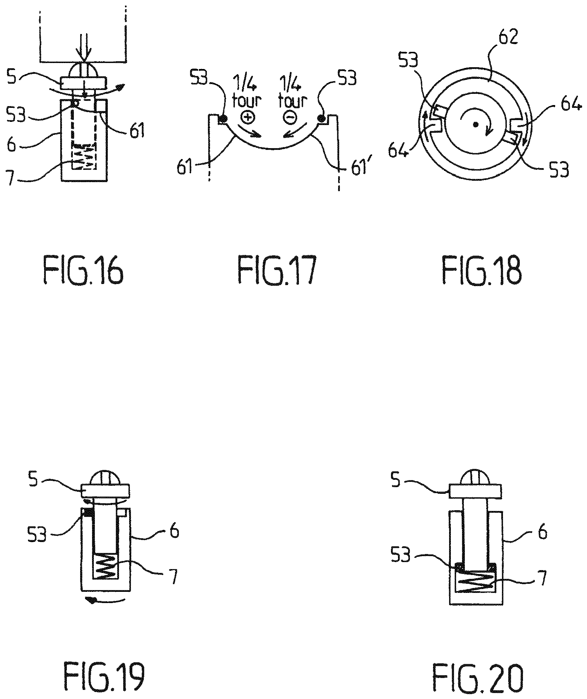

Les Figures 16 à 20 montrent un deuxième mode de réalisation selon l'invention. Le principe de ce mode est que l'entraineur 5 est guidé de sorte en plus à tourner sur lui-même quand il passe de la position de marche (PM) à la position escamotée (PE) jusqu'à ce que l'extrémité entrainante 51 se trouve face à la cavité 113 avant que l'entraineur 5 soit repoussé vers la position de marche (PM) par le ressort 7. Pour ce faire, l'extrémité entrainée 52 comprend sur sa surface latérale au moins un plot 53 destiné à entraîner en rotation l'entraîneur lorsque celui-ci est poussé vers la position escamotée (PE) lors de la mise en place de la tête de massage. L'évidement 62 de l'intermédiaire 6 présente une forme substantiellement cylindrique et au moins une rampe de guidage 61 formant une pente destinée à recevoir ledit plot 53 de telle sorte qu'il puisse glisser sur la pente vers l'intérieur de l'intermédiaire 6, c'est-à-dire vers le bas sur les Figures 16, 17, 19 et 20. La longueur de la rampe de guidage 61 projetée sur l'axe Δ est donc d'une distance D minimum.

On peut aussi imaginer, comme schématisé à la Figure 17, deux rampes de guidage 61, 61', symétriques et reliées par le bas de leurs pentes respectives. Ceci permet à l'entraineur 5 de pouvoir tourner dans un sens ou dans l'autre grâce au guidage du plot 53.

En fonction des symétries de forme que présente l'extrémité entrainante 51, il est également possible d'adapter le nombre de plots, ainsi que le nombre de rampes de guidage 61 et leur longueur. Par exemple, pour une extrémité entraînante 51 en forme de croix, c'est-à-dire composée de quatre portions identiques, l'extrémité entrainée 52 peut comporter deux plots 53 répartis de façon homogène et l'intermédiaire 6 quatre rampes de guidage 61, symétriques deux à deux comme schématisé à la Figure 17, pour que l'entraineur 5 tourne au maximum d'un quart de tour, dans un sens ou dans l'autre, avant d'être en face de la tête de massage.

Un ressort 7 est toujours disposé entre l'entraineur 5 et l'intermédiaire 6 pour pousser le premier vers la position de marche (PM). Selon l'exemple illustré, le ressort 7 est agencé dans l'évidement 62 de l'intermédiaire avec une extrémité en appui sur le fond de l'évidement 62 et l'autre sur l'extrémité entrainée 52.

La Figure 18 explique schématiquement l'entrainement de l'entraineur 5 en position de marche (PM). L'intermédiaire 6 comprend dans l'évidement 62 deux cales 64 en haut de la rampe de guidage 61 permettant d'entrer en contact avec lesdits plots 53 lorsque l'entraineur 5 est arrivé en position de marche (PM) et d'entrainer celui-ci en rotation. Selon l'exemple illustré, deux plots 53 et deux cales 64 sont utilisés pour une meilleure symétrie et répartition des efforts lors de l'entraînement. La Figure 19 illustre une première variante de ce mode de réalisation, avec les plots 53 en haut de l'extrémité entrainée 52 ; la Figure 20 illustre une deuxième variante avec les plots 53 en bas de l'extrémité entrainée 52.

En fonctionnement, lors de la mise en place d'une tête de massage 11, 12, ou 13 sur le corps creux 2, l'entraineur 5 est initialement en position de marche (PM). Si les formes complémentaires de l'extrémité entrainante 51 et de la cavité 113 ne sont pas en face, la tête de massage pousse l'entraineur 5 vers l'intérieur du corps creux 2 ; l'au moins un plot 53 glisse sur la pente de la rampe de guidage 61 et fait tourner légèrement l'entraineur jusqu'à ce que les formes complémentaires soient face à face ; sous l'effort de poussée du ressort 7, l'entraineur 5 se déplace vers la position de marche (PM) et s'emboîte avec la tête de massage mise en place avant d'entrainer les moyens de massage de celle-ci.

L'avantage de ce deuxième mode de réalisation est qu'il permet que le bruit éventuel dû à l'enclenchement de l'entraineur 5 dans la tête de massage survienne, non au moment de la mise en marche du moteur, mais au moment de la mise en place de la tête de massage sur le corps creux, ce qui paraitra plus logique à l'utilisateur et augmentera le confort d'utilisation de l'appareil.

Les Figures 14 et 15 montrent des alternatives qui pourraient être apportées à l'extrémité entrainante 51 dans tous les modes de réalisation. Par exemple, à la Figure 14, la forme de l'extrémité entrainante 51 ainsi que la forme correspondante de la cavité 113 peuvent être légèrement pyramidales et arrondies pour faciliter l'insertion de la partie mâle dans la partie femelle et la rendre plus progressive quand le moteur commence à tourner. Ceci permet de diminuer le bruit lors de l'enclenchement. La forme peut également présenter un coté arrondi pour l'insertion et l'autre coté plat pour un entrainement efficace, tel que visible à la Figure 15. Les cotés arrondis et les cotés plats peuvent être respectivement symétriques par rapport au plan central de l'entraineur 5 permettant une rotation de celui-ci dans un sens ou dans l'autre.

Afin d'encore diminuer le bruit pendant le fonctionnement de l'appareil, un moyen d'amortissement en matériau souple peut être prévu entre la cavité 113 et l'extrémité entrainante 51. Comme illustré à la Figure 13, l'entraineur 5 comprend sur l'extrémité entrainante 51 un patin amortisseur 55. Alternativement ou additionnellement, le patin amortisseur 55 peut aussi se trouver dans la cavité 113 ou sur le plateau 112 de la tête de massage, ou encore sur le corps creux 2.

L'appareil peut comprendre d'autres fonctions intégrées au corps creux 2 ou dans une tête de massage.

Bien entendu, d'autres modifications peuvent être apportées à l'invention dans le cadre des revendications annexées.

## Revendications

1. Appareil de soin (1) pour la peau comprenant un
- corps creux (2) formant une zone de préhension (21) et un logement (22) prévu pour recevoir de façon détachable au moins une tête de massage (11, 12,13),
- ledit corps creux (2) comprenant un moteur électrique (M) destiné à entrainer un moyen d'entrainement (5) autour d'un axe Δ par l'intermédiaire d'un moyen de transmission mécanique (8),
- ladite au moins une tête de massage (11, 12, 13) étant destinée à être actionnée par ledit moyen d'entrainement (5),
**Caractérisé en ce que** :
le moyen de transmission mécanique (8) comprend un moyen de guidage (6) dudit moyen d'entrainement (5) en translation selon ledit axe Δ entre au moins deux positions distinctes, dont une position de marche (PM) et une position escamotée (PE) dans laquelle ledit moyen d'entrainement (5) ne dépasse pas dans ledit logement (22) et ne gêne ainsi pas la mise en place de la tête de massage.

2. Appareil de soin selon la revendication précédente, **caractérisé en ce qu'**il comprend un moyen de rappel (7) actif entre le moyen d'entrainement (5) et le moyen de guidage (6) destiné à ramener le moyen d'entrainement (5) en position de marche (PM).

3. Appareil de soin selon la revendication 1 ou 2, **caractérisé en ce que** ledit moyen d'entrainement (5) présente une extrémité entrainante (51, 51', 51") destinée à entrer en contact avec ladite tête de massage (11, 12, 13) et une extrémité entrainée (52) destinée à rester en contact avec ledit moyen de guidage (6).

4. Appareil de soin selon la revendication précédente, **caractérisé en ce que** la tête de massage (11, 12, 13) comprend une connexion mécanique (113) présentant une forme complémentaire à la forme de ladite extrémité entrainante (51, 51', 51").

5. Appareil de soin selon la revendication précédente, **caractérisé en ce que** ladite extrémité entrainante (51, 51', 51") présente une forme saillante par rapport au moyen d'entrainement (5) et que la connexion mécanique (113) présente une forme creuse par rapport à la tête de massage (11, 12, 13).

6. Appareil de soin selon l'une des revendication précédentes, **caractérisé en ce que** l'extrémité entrainée (52) présente une forme substantiellement cylindrique et comprend sur la surface latérale au moins un plot (53) destiné à se déplacer entre la position de marche (PM) et la position escamotée (PE) sur au moins une rampe de guidage (61) appartenant au moyen de guidage (6).

7. Appareil de soin selon la revendication précédente, **caractérisé en ce que** le moyen de guidage (6) comprend au moins une cale (64) destinée à entrer en contact avec ledit plot (53) et à pouvoir entrainer celui-ci en rotation dans un sens comme dans l'autre lorsque le moyen d'entrainement (5) est en position de marche (PM).

8. Appareil de soin selon l'une des revendications précédentes, **caractérisé en ce qu'**il comprend des moyens d'indexage (23, 123) entre la tête de massage (11, 12, 13) et le corps creux (2).

9. Appareil de soin selon l'une des revendications précédentes, **caractérisé en ce qu'**il comprend des moyens de fixation (24, 124) par clips entre la tête de massage (11, 12, 13) et le corps creux (2).

10. Appareil de soin selon l'une des revendications précédentes, **caractérisé en ce qu'**il comprend un moyen d'amortissement (55) sur l'extrémité entrainante (51) du moyen d'entrainement (5).

11. Appareil de soin selon l'une des revendications précédentes, **caractérisé en ce que** le corps creux (2) est d'une forme longitudinale selon l'axe Δ.

12. Appareil de soin selon l'une des revendications précédentes, **caractérisé en ce qu'**il comprend au moins une source lumineuse (27) destinée à irradier la peau.

## Patentansprüche

1. Pflegevorrichtung (1) für die Haut, umfassend einen
- Hohlkörper (2), der einen Griffbereich (21) und einen Aufnahmeraum (22) bildet, derart angeordnet, um mindestens einen Massagekopf (11, 12, 13) auf abnehmbare Weise aufzunehmen,
- wobei der Hohlkörper (2) einen Elektromotor (M) umfasst, der dazu bestimmt ist, mittels eines mechanischen Getriebemittels (8) ein Antriebsmittel (5) um eine Achse Δ anzutreiben,
- wobei der mindestens eine Massagekopf (11, 12, 13) dazu bestimmt ist, durch das Antriebsmittel (5) betätigt zu werden,
**dadurch gekennzeichnet, dass**:
das mechanische Getriebemittel (8) in Translationsrichtung ein Führungsmittel (6) des Antriebsmittels (5) entlang der Achse Δ zwischen mindestens zwei unterschiedlichen Positionen umfasst, davon eine Betriebsposition (PM) und eine Versenkposition (PE), in der das Antriebsmittel (5) nicht in den Aufnahmeraum (22) hervorsteht und somit das Einsetzen des Massagekopfes nicht stört.

2. Pflegevorrichtung nach dem vorstehenden Anspruch, **dadurch gekennzeichnet, dass** sie ein Rückstellmittel (7) umfasst, aktiv zwischen dem Antriebsmittel (5) und dem Führungsmittel (6), das dazu bestimmt ist, das Antriebsmittel (5) in die Betriebsposition (PM) zurückzubringen.

3. Pflegevorrichtung nach Anspruch 1 oder 2, **dadurch gekennzeichnet, dass** das Antriebsmittel (5) ein antreibendes Ende (51, 51', 51"), das dazu bestimmt ist, mit dem Massagekopf (11, 12, 13) in Kontakt zu treten, und ein angetriebenes Ende (52) aufweist, das dazu bestimmt ist, mit dem Führungsmittel (6) in Kontakt zu bleiben.

4. Pflegevorrichtung nach dem vorstehenden Anspruch, **dadurch gekennzeichnet, dass** der Massagekopf (11, 12, 13) eine mechanische Verbindung (113) umfasst, die eine komplementäre Form zu der Form des antreibenden Endes (51, 51', 51") aufweist.

5. Pflegevorrichtung nach dem vorstehenden Anspruch, **dadurch gekennzeichnet, dass** das antreibende Ende (51, 51', 51") bezogen auf das Antriebsmittel (5) einen Vorsprung aufweist, und dass die mechanische Verbindung (113) bezogen auf den Massagekopf (11, 12, 13) eine Hohlform aufweist.

6. Pflegevorrichtung nach einem der vorstehenden Ansprüche, **dadurch gekennzeichnet, dass** das angetriebene Ende (52) eine im Wesentlichen zylindrische Form aufweist und auf der seitlichen Oberfläche mindestens einen Stift (53) umfasst, der dazu bestimmt, sich zwischen der Betriebsposition (PM) und der Versenkposition (PE) auf mindestens einer Nockenspur (61), die zu dem Führungsmittel (6) gehört, zu bewegen.

7. Pflegevorrichtung nach dem vorstehenden Anspruch, **dadurch gekennzeichnet, dass** das Führungsmittel (6) mindestens ein Abstandsstück (64) umfasst, das dazu bestimmt ist, mit dem Stift (53) in Kontakt zu treten, und um diesen in die eine wie die andere Richtung drehend antreiben zu können, wenn das Antriebsmittel (5) sich in Betriebsposition (PM) befindet.

8. Pflegevorrichtung nach einem der vorstehenden Ansprüche, **dadurch gekennzeichnet, dass** sie Indexierungsmittel (23, 123) zwischen dem Massagekopf (11, 12, 13) und dem Hohlkörper (2) umfasst.

9. Pflegevorrichtung nach einem der vorstehenden Ansprüche, **dadurch gekennzeichnet, dass** sie Befestigungsclipsmittel (24, 124) zwischen dem Massagekopf (11, 12, 13) und dem Hohlkörper (2) umfasst.

10. Pflegevorrichtung nach einem der vorstehenden Ansprüche, **dadurch gekennzeichnet, dass** sie ein Dämpfungsmittel (55) an dem antreibenden Ende (51) des Antriebsmittels (5) umfasst.

11. Pflegevorrichtung nach einem der vorstehenden Ansprüche, **dadurch gekennzeichnet, dass** der Hohlkörper (2) entlang der Achse Δ von einer länglichen Form ist.

12. Pflegevorrichtung nach einem der vorstehenden Ansprüche, **dadurch gekennzeichnet, dass** sie mindestens eine Lichtquelle (27) umfasst, die dazu bestimmt ist, die Haut zu beleuchten.

## Claims

1. Care apparatus (1) for skin comprising a
- hollow body (2) forming a gripping zone (21) and a housing (22) provided to detachably receive at least one massage head (11, 12, 13),
- said hollow body (2) comprising an electric motor (M) intended to drive a drive means (5) around an axis Δ by way of a mechanical transmission means (8),
- said at least one massage head (11, 12, 13) being intended to be actuated by said drive means (5),
**Characterised in that**:
the mechanical transmission means (8) comprises a means for guiding (6) said drive means (5) in translation about said axis Δ between at least two separate positions, of which one working position (PM) and one retracted position (PE) wherein said drive means (5) does not protrude in said housing (22) and does not thus impede the implementation of the massage head.

2. Care apparatus according to the preceding claim, **characterised in that** it comprises an active return means (7) between the drive means (5) and the guiding means (6) intended to bring back the drive means (5) into working position (PM).

3. Care apparatus according to claim 1 or 2, **characterised in that** said drive means (5) has a driving end (51, 51', 51") intended to enter into contact with said massage head (11, 12, 13) and a driven end (52) intended to remain in contact with said guiding means (6).

4. Care apparatus according to the preceding claim, **characterised in that** the massage head (11, 12, 13) comprises a mechanical connection (113) having a shape that is complementary to the shape of said driving end (51, 51', 51").

5. Care apparatus according to the preceding claim, **characterised in that** said driving end (51, 51', 51") has a shape protruding with respect to the drive means (5) and that the mechanical connection (113) has a hollow shape with respect to the massage head (11, 12, 13).

6. Care apparatus according to one of the preceding claims, **characterised in that** the driven end (52) has a substantially cylindrical shape and comprises, on the side surface, at least one platform (53) intended to be moved between the working position (PM) and the retracted position (PE) on at least one guiding rail (61) belonging to the guiding means (6).

7. Care apparatus according to the preceding claim, **characterised in that** the guiding means (6) comprises at least one wedge (64) intended to enter into contact with said platform (53) and to be able to drive it in rotation in one direction like in the other, when the drive means (5) is in working position (PM).

8. Care apparatus according to one of the preceding claims, **characterised in that** it comprises indexing means (23, 123) between the massage head (11, 12, 13) and the hollow body (2).

9. Care apparatus according to one of the preceding claims, **characterised in that** it comprises means for securing (24, 124) through clips between the massage head (11, 12, 13) and the hollow body (2).

10. Care apparatus according to one of the preceding claims, **characterised in that** it comprises a damping means (55) on the driving end (51) of the drive means (5).

11. Care apparatus according to one of the preceding claims, **characterised in that** the hollow body (2) has a longitudinal shape about the axis Δ.

12. Care apparatus according to one of the preceding claims, **characterised in that** it comprises at least one light source (27) intended to irradiate the skin.
